**Europäisches Patentamt**

**European Patent Office**

**Office européen des brevets**

(19)

(11) Numéro de publication : **0 549 420 A1**

# DEMANDE DE BREVET EUROPEEN

(12)

(21) Numéro de dépôt : **92403437.4**

(22) Date de dépôt : **17.12.92**

(51) Int. Cl.$^5$ : **A61K 35/78, A61K 47/40, A61K 47/26, A61K 9/20**

(30) Priorité : **23.12.91 FR 9115983**

(43) Date de publication de la demande :
**30.06.93 Bulletin 93/26**

(84) Etats contractants désignés :
**AT BE CH DE ES FR GB IT LI LU NL**

(71) Demandeur : **LABORATOIRES ARDEVAL**
**29 rue Marcel Cachin**
**F-94204 Ivry sur Seine (FR)**

(72) Inventeur : **Derrieu, Guy**
**Le Riviera Parc, 33 Chemin du Lautin**
**F-06800 Cagnes sur Mer (FR)**
Inventeur : **Raynier, Bernard Azur Sea**
**98 Corniche Fleurie**
**F-06200 Nice (FR)**

(74) Mandataire : **Orès, Bernard et al**
**Cabinet ORES 6, Avenue de Messine**
**F-75008 Paris (FR)**

(54) **Forme galénique sèche et poreuse à base de plantes, son procédé de préparation et ses applications.**

(57)    Compositions solides à base de plantes, aptes à être mises sous une forme galénique sèche et poreuse (lyophilisée ou équivalente), unitaire ou pulvérisée.

Ladite forme galénique sèche et poreuse comprend une composition solide II constituée par
**. une composition solide I composée de :**
— (A) un ou plusieurs principes actifs végétaux, inclus dans une suspension homogène de plantes fraîches ou sèches, obtenue par (a) nettoyage et/ou séchage de la plante ou partie de plante, (b) cryobroyage à une température inférieure à 0°C jusqu'à l'obtention d'une granulométrie inférieure à environ 100 µm, (c) trempage des particules pendant un temps convenable dans au moins un solvant approprié, (d) séparation de la phase liquide et de la phase solide de la suspension obtenue, (e) expression de la phase solide obtenue en (d) par pressage à froid, (f) mélange de la phase liquide obtenue en (d) et du pressat obtenu en (e), et
— (B) au moins un promoteur d'absorption à action séquestrante, lequel mélange (A + B) est séché par élimination de solvant ; et
**. (C) au moins un promoteur d'absorption choisi parmi les agents tensioactifs,**
laquelle composition II est lyophilisée et éventuellement associée à des excipients convenables d'un point de vue pharmaceutique.

EP 0 549 420 A1

La présente invention est relative à une composition solide à base de plantes entières et/ou de parties de plantes fraîches et/ou sèches et/ou d'extraits, apte à être mise sous une forme galénique sèche et poreuse (lyophilisée ou équivalente), unitaire ou pulvérisée ; une telle forme galénique est particulièrement bien adaptée à l'accroissement de la stabilité et de l'absorption de tous les principes actifs de plantes entières et/ou de parties de plantes fraîches et/ou sèches et/ou d'extraits.

L'invention est également relative au procédé de préparation de ladite composition sous forme sèche et de ladite forme galénique poreuse, à base de plantes.

Les Brevets français 2 036 890, 2 366 835 et 2 647 343 décrivent des formes pharmaceutiques ayant pour caractéristique de se dissoudre ou de se désintégrer rapidement en milieu aqueux ou dans la salive.

Toutefois, ces Brevets ne sont pas adaptés au traitement des plantes, et notamment à l'obtention de compositions à base de plantes sous forme solide, à absorption accrue, et conduisant à une biodisponibilité nettement améliorée des principes actifs. En effet, la composition chimique des principes actifs d'une plante et/ou partie de plante est très complexe ; les molécules de base, les génines d'hétérosides, dérivés terpéniques, triterpéniques, alcaloïdes, produits phénoliques, produits volatils divers, etc..., se présentent associées avec d'autres molécules en particulier des sucres, ce qui leur confèrent des poids moléculaires très différents allant de 100-200 à plusieurs milliers de daltons; ces hétérosides sont très sensibles aux dégradations aussi bien dues aux différents traitements qu'ils peuvent subir lors de la préparation de la composition que lors de leur administration.

La Demande de Brevet européen 0 420 729 décrit une technique de préparation conduisant à la stabilisation de plantes entières et/ou de parties de plantes fraîches ou sèches ou de leurs extraits, qui permet d'obtenir de manière stable en milieu liquide, principalement alcoolique, le *totum* du matériel végétal de départ stabilisé.

Cette Demande EP 0 420 729 décrit également l'obtention, par opération physique ou absorption sur un support, d'une composition solide. Cependant, une telle composition solide présente un certain nombre d'inconvénients :

. le pourcentage de support doit être nettement supérieur à celui de la suspension de plantes, pour avoir effectivement un pouvoir absorbant,

. la composition solide ainsi obtenue contient des solvants qui sont incompatibles avec la mise en forme de cette composition sous une forme galénique poreuse (lyophilisat, notamment),

. les principes actifs végétaux des formes solides décrites dans cette Demande ne présentent pas une biodisponibilité optimale.

Il était donc particulièrement intéressant de pouvoir disposer d'une forme galénique sèche à base de plantes, stable et permettant l'absorption de tous les principes actifs, sans dégradation de ces derniers, notamment au moment de l'absorption (amélioration de la biodisponibilité).

Poursuivant ses travaux, la Demanderesse a trouvé qu'il était possible d'obtenir une forme galénique sèche et poreuse à partir d'une composition solide à base de plantes, qui soit stable aussi bien aux dégradations chimiques, enzymatiques, bactériennes ou fongiques dues à la préparation et dont l'absorption au niveau des muqueuses soit significativement augmentée.

La présente invention s'est par conséquent donné pour but de pourvoir à une composition sous forme solide à base de plantes entières et/ou de parties de plantes, apte à être mise sous une forme galénique sèche et poreuse et qui répond mieux aux nécessités de la pratique, notamment en ce qu'elle évite toutes les dégradations des principes actifs végétaux et permet une absorption de tous ces principes actifs au niveau perlingual, ce qui évite, de plus, leur dégradation dans le tube digestif.

La présente invention a pour objet une composition solide I, à base de plantes, caractérisée en ce qu'elle comprend en mélange, au moins :

- (A) un ou plusieurs principes actifs végétaux, inclus dans une suspension homogène de plantes fraîches ou sèches, obtenue par (a) nettoyage et/ou séchage de la plante ou partie de plante, (b) cryobroyage à une température inférieure à 0°C jusqu'à l'obtention d'une granulométrie inférieure à environ 100 μm, (c) trempage des particules pendant un temps convenable dans au moins un solvant approprié, (d) séparation de la phase liquide et de la phase solide de la suspension obtenue, (e) expression de la phase solide obtenue en (d) par pressage à froid, (f) mélange de la phase liquide obtenue en (d) et du pressat obtenu en (e), et

- (B) au moins un promoteur d'absorption à action séquestrante,

lequel mélange (A + B) est séché par élimination de solvant.

Selon un mode de réalisation avantageux de ladite composition, le promoteur d'absorption à action séquestrante est choisi parmi les cyclodextrines α,β,γ ou les cyclodextrines polymérisées ou substituées, par exemple par des radicaux méthyle, éthyle, hydroxyéthyle ou hydroxypropyle, les aminocyclodextrines et les maltodextrines.

Selon une disposition avantageuse de ce mode de réalisation, ladite composition comprend comme promoteur d'absorption à action séquestrante, une association de β-cyclodextrine et de maltodextrines.

La composition solide I a l'avantage de permettre l'immobilisation de tous les principes actifs végétaux présents, quelle que soit leur structure et leur poids moléculaire et permet une stabilisation accrue de l'ensemble desdits principes actifs ; de plus cette composition I sous forme solide présente également l'avantage de contenir une quantité minimale de solvants et d'être apte à être mise sous une forme galénique sèche et poreuse, qui permet d'accroître encore l'absorption desdits principes actifs végétaux, au niveau des muqueuses.

Selon un autre mode de réalisation avantageux de ladite composition solide 1, à base de plantes, la quantité relative (en poids) des principes actifs par rapport au promoteur d'absorption à activité séquestrante varie dans des proportions allant de 5 à 50 %, de préférence entre 10 et 25-30 % en poids.

Selon un autre mode de réalisation avantageux de ladite composition I, ledit séchage est réalisé jusqu'à élimination complète des solvants organiques et élimination des solvants aqueux, de l'ordre de 90 %.

L'élimination des solvants peut être réalisée, notamment par une opération physique telle qu'évaporation, dessiccation, atomisation, filtration tangentielle.

De manière préférée, lorsque ladite élimination est réalisée par évaporation, celle-ci est réalisée sous pression réduite et est éventuellement associée à une exposition aux hyperfréquences.

La présente invention a également pour objet une composition solide II, à base de plantes, caractérisée en ce qu'elle comprend :
- une composition solide I, telle que définie ci-dessus (mélange de (A) + (B) séché) et
- (C) au moins un promoteur d'absorption choisi parmi les agents tensioactifs.

Selon un mode de réalisation avantageux de ladite composition II, le promoteur d'absorption de type tensioactif est choisi parmi l'acide glycyrrhizinique ou l'un de ses sels et le glycyrrhétinate de sodium.

Selon un autre mode de réalisation avantageux de ladite composition solide II, à base de plantes, la quantité de promoteur d'absorption de type tensioactif est comprise entre 0,5 et 5 % (p/p) et préférentiellement voisine de 1 % (p/p).

De manière surprenante, une telle composition sous forme solide II, à base de plantes est particulièrement apte à être mise sous une forme galénique sèche et poreuse (lyophilisée ou équivalente), qui présente une absorption accrue des principes actifs végétaux au niveau des muqueuses.

La présente invention a également pour objet une forme galénique sèche et poreuse, unitaire ou pulvérisée, caractérisée en ce qu'elle comprend une composition solide II lyophilisée, éventuellement associée à des excipients convenables d'un point de vue pharmaceutique.

De manière surprenante, une telle forme galénique sèche et poreuse, à base de plantes présente une absorption accrue des principes actifs végétaux car elle se dissout ou se désintègre rapidement en milieu aqueux (salive notamment) et facilite le passage de tous les principes actifs originels stabilisés des plantes et/ou parties de plantes, à travers les muqueuses (passage perlingual, par exemple).

L'utilisation d'adjuvants ou promoteurs d'absorption a été mentionnée dans de nombreuses publications ou Brevets. Par exemple pour les cyclodextrines, .elles accroissent l'absorption du cholécalciférol (DUCHENE et coll., S.T.P. Pharma, 1(1), 37-43 (1985)); des vitamines A, E, K (DUCHENE D.et coll., Labo Pharma Probl., 32(348), 842-850 (1984) et SZEJTLI J.et coll., Die Stärke, 32(11), 386-391 (1980)); pour le glycyrrhétinate de sodium, l'absorption nasale de l'insuline (MISHIMA et coll., J. Pharmacobio-Dyn., 10, s-69 (1987)); pour l'acide glycyrrhizinique et ses sels, l'absorption à travers une muqueuse de la calcitonine (Brevet français n° 2 623 090).

Cependant, la Demanderesse a trouvé, de manière surprenante, qu'associer un promoteur d'absorption tensioactif, en particulier l'acide glycyrrhizinique ou un de ses sels à un promoteur d'absorption à activité séquestrante (β-cyclodextrine, par exemple) entraîne une synergie de l'absorption des principes actifs associés, au niveau des muqueuses et plus particulièrement au niveau de la muqueuse buccale, laquelle synergie d'action est encore accrue lorsque ladite composition est poreuse (lyophilisation).

De manière avantageuse, ladite forme galénique sèche et poreuse à base de plantes comprend en outre des excipients convenables tels que des diluants, des liants, des agents de délitement ou désintégration et des agents de surface, éventuellement associés à des additifs facultatifs tels que des arômes, parfums ou édulcorants, des colorants, des conservateurs ou des correcteurs de pH.

De telles formes galéniques sèches et poreuses à base de plantes ont l'avantage à la fois :
. de conserver la totalité des principes actifs végétaux dans l'état où ils se trouvent dans la plante et/ou dans la partie de plante, et
. de présenter, du fait de l'association de deux promoteurs d'absorption de type différent, et de la mise sous une forme poreuse (lyophilisation, notamment) une absorption nettement améliorée au niveau des muqueuses, ce qui augmente l'efficacité desdits principes actifs, du fait de leur meilleure biodisponibilité.

Les diluants plus particulièrement utilisés dans les formes galéniques sèches et poreuses conformes à l'invention sont notamment des matières pharmaceutiquement acceptables, de préférence solubles, choisies notamment parmi le lactose, le mannitol, le glycocolle, le sorbitol, ou des mélanges de ces substances.

Les liants plus particulièrement utilisés dans les formes galéniques conformes à l'invention sont notamment toute matière soluble ou dispersible dans l'eau, à même d'assurer la cohésion desdites compositions, et inerte vis à vis des principes actifs. Ces matières sont choisies préférentiellement parmi les polysaccharides, tels que par exemple des gommes naturelles du type gomme arabique, les alginates, les pectines, la gélatine, des gommes synthétiques du type gomme xanthane, les dextranes, la dextrine, les dérivés de la cellulose, l'alcool polyvinylique, la polyvinylpyrrolidone, ou encore des mélanges de ces substances.

Les agents de délitement ou désintégration, les agents de surface, les arômes, les parfums ou édulcorants, les colorants, les conservateurs, les correcteurs de pH sont ceux habituellement utilisés dans l'industrie pharmaceutique et alimentaire pour l'élaboration de formes comparables et compatibles avec les principes actifs en présence.

La présente invention a également pour objet un procédé de préparation de la forme galénique sèche et poreuse conforme à l'invention, caractérisé en ce qu'il comprend les étapes suivantes :

1) préparation d'une suspension homogène (A) de plantes contenant un ou plusieurs principes actifs végétaux par (a) nettoyage et/ou séchage de la plante ou partie de plante, (b) cryobroyage à une température inférieure à 0°C jusqu'à l'obtention d'une granulométrie inférieure à environ 100 μm, (c) trempage des particules pendant un temps convenable dans au moins un solvant approprié, (d) séparation de la phase liquide et la phase solide de la suspension obtenue, (e) expression de la phase solide obtenue en (d) par pressage à froid, (f) mélange de la phase liquide obtenue en (d) et du pressat obtenu en (e) ;

2) immobilisation des principes actifs végétaux obtenus en 1) par mélange de la suspension (A) avec au moins un promoteur d'absorption à activité séquestrante (B) ;

3) élimination des solvants contenus dans le mélange obtenu en 2), pour l'obtention d'une composition I, conforme à l'invention ;

4) obtention d'une pâte à partir d'une composition II conforme à l'invention, par mélange du produit obtenu en 3) (composition I) avec une préparation contenant au moins un promoteur d'absorption de type tensioactif (C), éventuellement associé aux diluants, liants et aux additifs facultatifs ainsi qu'une quantité de liquide convenable ;

5) mise sous forme poreuse de la composition obtenue en 4), notamment par congélation et sublimation (lyophilisation) ou opération équivalente.

Selon un mode de mise en oeuvre avantageux dudit procédé, ladite élimination comprend l'élimination complète des solvants organiques et l'élimination des solvants aqueux, de l'ordre de 90 %.

Selon un autre mode de mise en oeuvre, l'élimination des solvants est précédée d'une concentration, par exemple par osmose.

De préférence, les principes actifs végétaux obtenus en milieu hydroalcoolique suivant la Demande de Brevet européen 0 420 729 sont immobilisés par addition du/des promoteurs d'absorption à activité séquestrante (coprécipitation).

Selon un autre mode de mise en oeuvre avantageux dudit procédé, la quantité relative (en poids) des principes actifs par rapport aux promoteurs d'absorption à activité séquestrante varie dans des proportions allant de 5 à 50 %, de préférence entre 10 et 25-30 % en poids.

Selon un autre mode de mise en oeuvre avantageux dudit procédé, la quantité de promoteur d'absorption de type tensioactif est comprise entre 0,5 et 5 % (p/p) et préférentiellement voisine de 1 % (p/p).

Les formes galéniques sèches et poreuses conformes à l'invention trouvent application dans le domaine pharmaceutique, vétérinaire, diététique, alimentaire ou cosmétique.

Outre les dispositions qui précèdent, l'invention comprend encore d'autres dispositions, qui ressortiront de la description qui va suivre, qui se réfère à des exemples de compositions et de mise en oeuvre du procédé, objets de la présente invention.

Il doit être bien entendu, toutefois, que ces exemples sont donnés uniquement à titre d'illustration de l'objet de l'invention dont ils ne constituent en aucune manière une limitation.

Exemple 1 :

Les trois préparations suivantes, issues d'une même solution hydroalcoolique de saule (écorce) obtenue selon la Demande de Brevet européen 0 420 729, ont été réalisées dans un mélangeur planétaire supportant une pression réduite et équipé d'un générateur d'hyperfréquences :

|  | ESSAI 1 | ESSAI 2 | ESSAI 3 |
|---|---|---|---|
| saule, équivalent en plante sèche à | 50 g | 50 g | 50 g |
| β-cyclodextrine | 25 g | - | 15 g |
| Maltodextrines | - | 25 g | 10 g |
| teneur en eau résiduelle | 6,3 % | 2,5 % | 4,1 % |

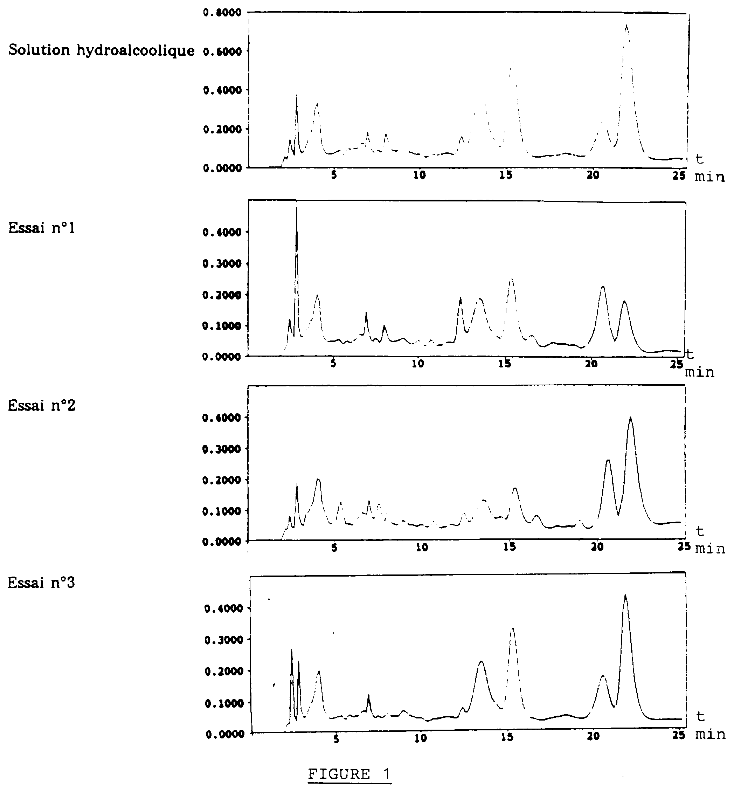

Une étude chromatographique par CLHP a été réalisée ; la comparaison des profils obtenus (figure 1) avec les trois essais, comparés à celui de la solution hydroalcoolique de départ, met en évidence la stabilisation de tous les composants du saule par le mélange des promoteurs d'absorption à activité séquestrante.

Exemple 2 :

Une préparation, issue de la solution hydroalcoolique de racines de bardane, réalisée selon l'exemple 1 de la Demande de Brevet européen 0 420 729, dont la composition est présentée dans le tableau ci-après, a été réalisée comme décrit précédemment à l'exemple 1 ci-dessus :

| COMPOSANTS | ESSAI |
|---|---|
| bardane, équivalente en plante sèche à | 50 g |
| β-cyclodextrine | 15 g |
| Maltodextrines | 10 g |
| teneur en eau résiduelle | 5,8 g |

Une étude analytique de la solution hydroalcoolique et de la préparation décrite ci-dessus a été réalisée. Les résultats sont exprimés par rapport à l'équivalent en racines séchées, comme visible dans le tableau ci-après.

| | SOLUTION | PREPARATION |
|---|---|---|
| PROTIDES | | |
| totaux | 1,8 % | 1,8 % |
| ac. aminés libres | 0,3 % | 0,4 % |
| ac. aminés après hydrolyse | 1,4 % | 1,4 % |
| SUCRES | | |
| totaux | 10,5 % | 11,6 % |
| inuline | 10 % | 11% |
| glucose | 0,180 % | 0,180 % |
| saccharose | 0,180 % | 0,170 % |
| DIVERS | | |
| lipides | traces | traces |
| vitamine E | 0,000015 % | 0,000014 % |
| flavonoïdes | 0,09 % | 0,12 % |
| stérols | 0,009 % | 0,0010 % |
| tanins | présence | présence |
| saponosides | présence | présence |

Il existe une forte similitude entre les deux analyses.

Exemple 3 :

Dans un mélangeur planétaire supportant une pression réduite et équipé d'un générateur d'hyperfréquences, on introduit la solution hydroalcoolique de saule (écorce) et les promoteurs d'absorption à activité séquestrante, β-cyclodextrine et maltodextrines. On mélange pendant 1 heure, puis on effectue l'élimination d'une partie du solvant sous vide à l'aide de micro-ondes (excitation en discontinu), en ne dépassant pas 35°C. L'opération est stoppée lorsque la quantité de solvant est voisine de 0,6 g par unité. On introduit alors tous les autres excipients selon la composition suivante (formule unitaire) :

| | |
|---|---|
| Solution hydroalcoolique de saule, équivalente en plante sèche à | 500 mg |
| β-cyclodextrine | 285 mg |
| Maltodextrines | 150 mg |
| Lactose | 920 mg |
| Gomme arabique | 33 mg |
| Glycyrrhizinate d'ammonium* | 15 mg |
| Aspartam | 11 mg |

* promoteur d'absorption de type tensioactif.

On mélange pendant 1 heure et on réalise des lyophilisats oraux de 1,5 g, de manière classique. Le temps de délitement varie de 2 à 4 minutes.

Exemple 4 :

On compare une préparation réalisée comme décrite précédemment à l'exemple 3, contenant de la β-cyclodextrine et du glycyrrhizinate d'ammonium comme promoteurs d'absorption, respectivement à activité séquestrante et de type tensioactif, dans un essai d'absorption par les muqueuses de la cavité buccale avec les préparations suivantes :

|  | ESSAI 1 (object de l'invention) | ESSAI 2 | ESSAI 3 | ESSAI 4 |
|---|---|---|---|---|
| Solution de racine d'ortie (en référence au β-sitostérol) | 5,2 mg | 5,2 mg | 5,2 mg | 5,2 mg |
| β-cyclodextrine | 285 mg | 285 mg | - | - |
| Maltodextrines | 150 mg | 150 mg | 150 mg | 150 mg |
| Glycyrrhizinate d'NH$_3$ | 15 mg | - | 15 mg | - |
| Gomme arabique | 33 mg | 33 mg | 33 mg | 33 mg |
| Lactose | 920 mg | 935 mg | 205 mg | 1220 mg |
| Aspartam | 11 mg | 11 mg | 11 mg | 11 mg |

L'absorption est déterminée par le test d'absorption buccale décrit par BECKETT A.H. et TRIGGS E.J., J. Pharm. Pharmacol., 19, 318-418, 1967. On donne à six volontaires deux lyophilisats oraux soit l'équivalent de 10,4 mg de β-sitostérol. On détermine le taux de β-sitostérol absorbé par les muqueuses, par dosage CPG.
Les résultats figurent dans le tableau suivant :

|  | Dose administrée en mg | Taux absorbé en mg |
|---|---|---|
| ESSAI 1 | 10,4 mg | 8,9 mg |
| ESSAI 2 | 10,4 mg | 4,6 mg |
| ESSAI 3 | 10,4 mg | 5,8 mg |
| ESSAI 4 | 10,4 mg | 1,8 mg |

L'essai 1, qui correspond à l'administration d'une composition conforme à l'invention, présente une absorption nettement améliorée par rapport aux autres essais.
Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention.

## Revendications

1°) Composition solide 1, à base de plantes, caractérisée en ce qu'elle comprend en mélange, au moins :
- (A) un ou plusieurs principes actifs végétaux, inclus dans une suspension homogène de plantes fraîches ou sèches, obtenue par (a) nettoyage et/ou séchage de la plante ou partie de plante, (b) cryobroyage à une température inférieure à 0°C jusqu'à l'obtention d'une granulométrie inférieure à environ 100 µm, (c) trempage des particules pendant un temps convenable dans au moins un solvant approprié, (d) séparation de la phase liquide et de la phase solide de la suspension obtenue, (e) expression de la phase solide obtenue en (d) par pressage à froid, (f) mélange de la phase liquide obtenue en (d) et du pressat obtenu en (e), et
- (B) au moins un promoteur d'absorption à action séquestrante,
lequel mélange (A + B) est séché par élimination de solvant.

2°) Composition solide I, à base de plantes, selon la revendication 1, caractérisée en ce que le promoteur d'absorption à action séquestrante est choisi parmi les cyclodextrines $\alpha,\beta,\gamma$ ou les cyclodextrines polymérisées ou substituées par exemple par des radicaux méthyle, éthyle, hydroxyéthyle ou hydroxypropyle, les aminocyclodextrines et les maltodextrines.

3°) Composition solide I, à base de plantes, selon la revendication 2, caractérisée en ce qu'elle comprend comme promoteur d'absorption à action séquestrante, une association de $\beta$-cyclodextrine et de maltodextrines.

4°) Composition solide I, à base de plantes, selon l'une quelconque des revendications 1 à 3, caractérisée en ce que la quantité relative (en poids) des principes actifs par rapport au promoteur d'absorption à activité séquestrante varie dans des proportions allant de 5 à 50 %, de préférence entre 10 et 25-30 % en poids.

5°) Composition I, à base de plantes, selon l'une quelconque des revendications 1 à 4, caractérisée en ce que ledit séchage est réalisé jusqu'à élimination complète des solvants organiques et élimination des solvants aqueux, de l'ordre de 90 %.

6°) Composition solide II, à base de plantes, caractérisée en ce qu'elle comprend :
- une composition solide 1 selon l'une quelconque des revendications 1 à 5, et
- (C) au moins un promoteur d'absorption choisi parmi les agents tensioactifs.

7°) Composition solide II, à base de plantes, selon la revendication 6, caractérisée en ce que le promoteur d'absorption de type tensioactif est choisi parmi l'acide glycyrrhizinique ou l'un de ses sels et le glycyrrhétinate de sodium.

8°) Composition solide II, à base de plantes, selon la revendication 6 ou la revendication 7, caractérisée en ce que la quantité de promoteur d'absorption de type tensioactif est comprise entre 0,5 et 5 % (p/p) et préférentiellement voisine de 1 % (p/p).

9°) Forme galénique sèche et poreuse, unitaire ou pulvérisée, caractérisée en ce qu'elle comprend une composition solide II selon l'une quelconque des revendications 6 à 8 lyophilisée, éventuellement associée à des excipients convenables d'un point de vue pharmaceutique.

10°) Procédé de préparation de la forme galénique sèche et poreuse selon la revendication 9, caractérisé en ce qu'il comprend les étapes suivantes :

1) préparation d'une suspension homogène (A) de plantes contenant un ou plusieurs principes actifs végétaux par (a) nettoyage et/ou séchage de la plante ou partie de plante, (b) cryobroyage à une température inférieure à 0°C jusqu'à l'obtention d'une granulométrie inférieure à environ 100 $\mu$m, (c) trempage des particules pendant un temps convenable dans au moins un solvant approprié, (d) séparation de la phase liquide et la phase solide de la suspension obtenue, (e) expression de la phase solide obtenue en (d) par pressage à froid, (f) mélange de la phase liquide obtenue en (d) et du pressat obtenu en (e) ;

2) immobilisation des principes actifs végétaux obtenus en 1) par mélange de la suspension (A) avec au moins un promoteur d'absorption à activité séquestrante (B) ;

3) élimination des solvants contenus dans le mélange obtenu en 2), pour l'obtention d'une composition I, selon l'une quelconque des revendications 1 à 5 ;

4) obtention d'une pâte à partir d'une composition II selon l'une quelconque des revendications 6 à 8, par mélange du produit obtenu en 3) (composition I) avec une préparation contenant au moins un promoteur d'absorption de type tensioactif (C), éventuellement associé aux diluants, liants et aux additifs facultatifs ainsi qu'une quantité de liquide convenable ;

5) mise sous forme poreuse de la composition obtenue en 4), notamment par congélation et sublimation (lyophilisation) ou opération équivalente.

11°) Procédé selon la revendication 10, caractérisé en ce que l'élimination comprend l'élimination complète des solvants organiques et l'élimination des solvants aqueux, de l'ordre de 90 %.

12°) Procédé selon la revendication 10 ou la revendication 11, caractérisé en ce que l'élimination des solvants est précédée d'une concentration, par exemple par osmose.

13°) Procédé selon l'une quelconque des revendications 10 à 12, caractérisé en ce que la quantité relative (en poids) des principes actifs par rapport aux promoteurs d'absorption à activité séquestrante varie dans des proportions allant de 5 à 50 %, de préférence entre 10 et 25-30 % en poids.

14°) Procédé selon l'une quelconque des revendications 10 à 13, caractérisé en ce que la quantité de promoteur d'absorption de type tensioactif est comprise entre 0,5 et 5 % (p/p) et préférentiellement voisine de 1 % (p/p).

15°) Application de la forme galénique sèche et poreuse selon la revendication 9 à l'usage pharmaceutique, vétérinaire, diététique, alimentaire ou cosmétique.

FIGURE 1

9

Office européen
des brevets

**RAPPORT DE RECHERCHE EUROPEENNE**

Numero de la demande

EP  92 40 3437

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| D,X<br>Y | EP-A-0 420 729 (LABORATOIRES ARDEVAL)<br><br>* revendications 1,7,11 *<br>* page 3, ligne 3 - ligne 12 *<br>* page 3, ligne 31 - ligne 37 *<br>* page 4, ligne 13 - ligne 18 *<br>--- | 1-6<br>7-15 | A61K35/78<br>A61K47/40<br>A61K47/26<br>A61K9/20 |
| Y | EP-A-0 397 447 (ISF SOCIETA PER AZIONI)<br>* revendications 1,4 *<br>* page 2, ligne 51 - ligne 54 *<br>* page 3, ligne 30 - ligne 32 *<br>* page 4, ligne 28 - ligne 33 *<br><br>----- | 7-15 | |

**DOMAINES TECHNIQUES RECHERCHES (Int. Cl.5 )**

A61K

Le présent rapport a été établi pour toutes les revendications

| Lieu de la recherche | Date d'achèvement de la recherche | Examinateur |
|---|---|---|
| LA HAYE | 03 FEVRIER 1993 | VENTURA AMAT A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

&  : membre de la même famille, document correspondant

EPO FORM 1503 03.82 (P0402)